(19) Europäisches Patentamt
European Patent Office
Office européen des brevets

(11) Veröffentlichungsnummer: **0 194 427**
**A2**

(12) # EUROPÄISCHE PATENTANMELDUNG

(21) Anmeldenummer: **86100976.9**

(22) Anmeldetag: **24.01.86**

(51) Int. Cl.[4]: **C 07 D 251/18**
**C 07 C 47/058**

(30) Priorität: **14.03.85 DE 3509056**

(43) Veröffentlichungstag der Anmeldung:
**17.09.86 Patentblatt 86/38**

(84) Benannte Vertragsstaaten:
**AT BE DE FR GB IT NL SE**

(71) Anmelder: **Degussa Aktiengesellschaft**
**Weissfrauenstrasse 9**
**D-6000 Frankfurt am Main 1(DE)**

(72) Erfinder: **Werle, Peter, Dr., Dipl.-Chem.**
**Im Börner 43**
**D-6460 Gelnhausen(DE)**

(72) Erfinder: **Focke, Holger, Dr., Dipl.-Chem.**
**Erlenweg 28**
**D-6454 Bruchköbel(DE)**

(72) Erfinder: **Boes, Alwin**
**Spechtstrasse 5**
**D-6078 Neu-Isenburg(DE)**

(54) **Verfahren zur Herstellung von Dodecamethylenbismelamin.**

(57) Die Erfindung betrifft ein Verfahren zur Herstellung von Dodecamethylenbismelamin durch Umsetzung von 2,4-Diamino-6-chlortriazin-(1,3,5) mit 1,12-Diaminododecan in einer alkalischen wässrigen Suspension, der man das Diamin in einem mit Wasser mischbaren Lösungsmittel zugesetzt hat.

Das Produkt wird zur Stabilisierung von wässrigen Formaldehydlösungen verwendet.



Croydon Printing Company Ltd.

D e g u s s a Aktiengesellschaft
Frankfurt am Main

# Verfahren zur Herstellung von Dodecamethylenbismelamin und dessen Verwendung

Die Erfindung betrifft die Herstellung von Dodecamethylenbismelamin und dessen Verwendung zur Stabilisierung von wässrigen Formaldehydlösungen.

Dodecamethylenbismelamin mit der Strukturformel

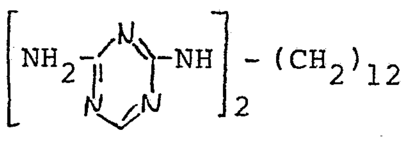

(I)

wird als Stabilisator für wässrige Formaldehydlösungen eingesetzt und kann gemäß der EP-A2 79037 durch Umsetzung einer wässrigen Aufschlämmung von 2,4-Diamino-6-chlortriazin-(1,3,5) mit 1,12 Diaminododecan in Gegenwart äquivalenter Mengen Natriumhydroxid erhalten werden. Dieses Verfahren liefert ein Produkt wechselnder Zusammensetzungen und oftmals nicht befriedigender Eigenschaften hinsichtlich seiner Eignung als Stabilisator.

Versucht man darüberhinaus eine Darstellung im technischen Maßstab, so wird das gewünschte Bismelamin überhaupt nur im Gemisch mit dem 1:1 Addukt und anderen Produkten erhalten und ist somit nicht brauchbar.

Die in der DE-OS 26 25 399 angegebene Herstellung von Solvaten des Bismelamins durch Umsetzung von 2,4 Diamino-6-chlor-s-triazin mit Diamin in Dimethylformamid führt zu feinverteilten wässrigen DMF-haltigen Suspensionen aus denen das Produkt nur sehr schwer als hellbraunes, wahrscheinlich dimethylformamidhaltiges Präparat von Schmelzpunkt 110 °C abgetrennt werden kann. Demgegenüber liegt der Schmelzpunkt des reinen Bismelamins mit der Formel (I) bei 186 - 188 °C. Auch ist die Stabilisierwirkung gegenüber Formaldehylösungen nicht ausreichend.
Gemäß US-PS 2 544 071 gewinnt man Decamethylendiamin aus der Umsetzung einer wässrigen Suspension von 2,4-Diamino-6-chlor-triazin-(1,3,5) mit Decamethylendiamin nur als Rohprodukt, dessen Verunreinigungen einen weiteren Reinigungsschritt notwendig machen.

Aufgabe der Erfindung ist, ein Dodecamethylenbismelamin herzustellen, das zur Stabilisierung von wässrigen Formaldehydlösungen besser geeignet ist.

Gegenstand der Erfindung ist ein Verfahren zur Herstellung von Dodecamethylenbismelamin durch Umsetzung von 2,4-Diamino-6-chlor-triazin-(1,3,5) mit 1,12-Diaminododecan in einer alkalischen wässrigen Suspension, das dadurch gekennzeichnet ist, daß man 1,12-Diaminododecan in einem mit Wasser mischbaren, organischen Lösungsmittel, insbesondere einem aliphatischen Alkohol, Keton, Ether oder Etheralkohol, gelöst der Suspension zusetzt, nach Ablauf der Reaktion das Reaktionsgemisch auf Raumtemperatur (20 - 25 °C) abkühlt und das auskristallisierte Produkt, das in ausgezeichnet filtrierbarer Form anfällt, abtrennt. Durch Zusatz von wässriger, bevorzugt 20 bis 30%iger Natronlauge wird gewährleistet, daß der pH-Wert während der Reaktion 10 nicht übersteigt und bevorzugt im Bereich von 8 bis 10 liegt.

Die Reaktanden werden im molaren Verhältnis von 1:1 eingesetzt, gegebenenfalls mit einem Überschuß von 5 bis zu 10 % 1,12-Diaminododecan. Geeignete Alkohole besitzen 1 - 3 Kohlenstoffatome; bevorzugt setzt man Methanol, Ethanol, n-Propanol oder Isopropanol oder aber Mischungen dieser Alkohole ein.
Weitere geeignete Lösungsmittel sind z.B. Dioxan, Ethylenglykolmonomethylether, Aceton, Methylethylketon, Tetrahydrofuran.

Die Menge an organischem Lösungsmittel sollte möglichst klein gehalten werden. In jedem Fall ist aber soviel einzusetzen, daß das Diamin vollständig gelöst der Reaktionsmischung zugesetzt werden kann.
Man arbeitet mit einem 3-8-fachen, bevorzugt 4-6-fachen Überschuß des Lösungsmittels, bezogen auf das Gewicht des Diamins.
Das Mengenverhältnis (Gew.-Teile) von Wasser zu organischem Lösungsmittel im Reaktionsgemisch beläuft sich auf 3:1 bis 10:1, bevorzugt 5:1 bis 7:1, während Wasser und Triazinverbindung in der wässrigen Suspension im Verhältnis von 90:10 bis 97:3 vorliegen, bevorzugt von 93:7 bis 95:5
Nach dem erfindungsgemäßen Verfahren kann man auch wässrige Supensionen einsetzen, die man aus der Umsetzung von Cyanurchlorid mit Ammoniak und Natriumhydroxid nach der Entfernung des überschüssigen Ammoniaks erhalten hat.

Wesentlich für die Auswahl eines geeigneten organischen Lösungsmittels ist auch, daß das gebildete Dodecamethylenbismelamin nach dem Abkühlen des Reaktionsgemisches auf Raumtemperatur auskristalliert..

Das erfindungsgemäße Verfahren liefert ein Produkt, das ohne weitere sonst notwendige Reinigungsschritte eine verbesserte Stabilisierwirkung in wässrigen Formaldehydlösungen zeigt.
Diese Wirkung kann man um einen weiteren positiven Effekt ergänzen, wenn man das Bismelamin nicht in fester Form, sondern in einer semiformalhaltigen Lösung den zu stabilisierenden Lösungen zusetzt.
Man erhält dann stabilisierte Formaldehydlösungen, die weitaus weniger schäumen.

Diese vorteilhafte Wirkung ist auch dann zu registrieren, wenn man Dodecamethylenbismelamin einsetzt, das nach dem Stand der Technik hergestellt wurde.

Dodecamethylenbismelamin ist in Methylsemiformal im Gegensatz zu gewöhnlichen organischen Lösungsmitteln auch bei Raumtemperatur so gut löslich, daß man leicht 20 - 25 Gew.%-ige Lösungen herstellen kann.
Dies ist mit dem zusätzlichen Vorteil verbunden, daß eventuell vorhandene Verunreinigungen in Form von chlorhaltigen Triazinderivaten leicht abgetrennt werden können, das sie in Methylsemiformal unlöslich sind.

Zur Herstellung einer semiformalhaltigen Stabilisatorlösung suspendiert man Paraformaldehyd in Methanol und erhitzt nach Zugabe eines organischen Amins als Katalysator zum Sieden, bis man eine klare Lösung erhält.

Dieser setzt man 15 bis 25 Gew.%, bevorzugt 20 bis 22 Gew.%, bezogen auf die Gesamtmenge, des Dodecamethylenbismelamins zu, rührt weitere 20 bis 30 Minuten, filtriert gegebenenfalls vorhandene unlösliche Bestandteile ab und erhält nach dem Erkalten eine gebrauchsfertige Stabilisatorlösung.

Als Lösungsmittel verwendet man bevorzugt Methoxymethanol in Form einer methanolischen Lösung, die 70 bis 85 Gew.-% Methoxymethanol enthält.
Als Amin werden bevorzugt Triethylamin, Diethylamin, Pyridin, Hexamethylentetramin in einer Menge von 0,1 bis 1,5 Gew.-%, bezogen auf das zu lösende Paraformaldehyd, verwendet. Wesentlich für die Wirkung der Amine ist die alkalische Einstellung. Auf die Stabilisierung üben sie keinen Einfluß aus. Zur Stabilisierung eignen sich Zusätze von Dodecamethylenbismelamin in Hähe von 0,001 bis 0,05 Gew.-%, bevorzugt 0,005 bis 0,015 Gew.-%, bezogen auf die Gesamtmenge der Formaldehydlösung.
Weitaus geringer schäumende stabilisierte Formaldehydlösungen erhält man auch bei Verwendung von Dodecamethylenbismelamin, das nach den Verfahren gemäß Stand der Technik hergestellt und in Methoxymethanol gelöst, eingesetzt wird.
Die Verwendung von Methanol zur Herstellung des Semiformals ist vorteilhaft, weil die Formaldehydlösung sowieso Methanol enthält. Prinzipiell können jedoch auch andere wasserlösliche Alkohole eingesetzt werden.

Herstellung von Dodecamethylenbismelamin

Beispiel 1.

Eine wässrige Suspension, die etwa 150 l Wasser und 14,6 kg 2,4-Diamino-6-Chlor-s-triazin enthält, wird auf 60° C erwärmt, anschließend eine Lösung von 10 kg 1,12-Diaminododecan in 50 l n-Propanol zugegeben und unter Rühren auf Rückflußtemperatur erwärmt. Durch Zugabe von 20 Gew. %iger Natronlauge etwa innerhalb von 2 h wird ein pH-Bereich von 8-10 aufrechterhalten. Wenn der pH-Wert sich nicht mehr ändert, wird noch 1-2 h weitergerührt, anschließend auf etwa 20° C abgekühlt und filtriert. Nach Auswaschen mit Wasser und trocknen erhält man ein völlig farbloses Produkt mit einem Schmelzpunkt von 186-188° C.
Ausbeute: 19,2 kg (94% d.Th.).

Das Bismelamin wurde anhand IR- und NMR- spektroskopischer Untersuchungen identifiziert.

Beispiel 2

Die Versuchsdurchführung erfolgt analog Beispiel 1, jedoch werden als Lösungsmittel für das Diamin 50 l Ethanol eingesetzt. Man erhält 19,4 kg (95% d.Th.) rein weisses, in Form kleiner Granulen vorliegendes Bismelamin.

Beispiel 3

Die Versuchsdurchführung erfolgt analog Beispiel 1, jedoch

werden als Lösungsmittel für das Diamin 45 l Ethylenglykolmonomethylether eingesetzt. Man erhält 19,0 kg (93% d.Th.) Bismelamin.

Stabilisierung der Formaldehyd-Lösungen

Beispiel 4

Es wurden Formaldehydlösungen mit verschiedenen Gehalten an Formaldehyd untersucht, denen zur Stabilisierung verschiedene Mengen des erfindungsgemäß hergestellten Bismelamins zugesetzt worden waren.

Den Tabellen 1 und 2 sind ebenfalls Ergebnisse von Versuchen mit Bismelamin, die gemäß der EP-A2-79037 bzw. der DE-OS 26 25 399 hergestellt wurden, zu entnehmen. Zur Auflösung der Stabilisatoren in den Formaldehyd-Lösungen wurden diese etwa 30 Minuten lang unter Rühren auf 80° C gehalten. Die Stabilisatorgehalte sind in Gewichtsprozenten, bezogen auf die Gesamt-Formaldehyd-Lösung angegeben. Als Lagerhaltbarkeit gilt die Zeit, in der die Lösung stabil ist. Sie endet, sobald die erste wahrnehmbare Abscheidung an Paraformaldehyd auftritt.

Tabelle 1

Lösungen mit 37 Gew. % Formaldehyd und 0,40 Gew. % Methanol

| Stabilisator (Dodecamethylen-bismelamin) | Gehalt (%) | Lagertemperatur (K) | Lagerhaltbarkeit (d) |
|---|---|---|---|
| gem. Erfindung | 0,005 | 273 | > 60 |
| | 0,01 | 273 | >120 |
| gem. EP-A2-79037 | 0,005 | 273 | 6 |
| | 0,01 | 273 | 28 |
| gem. DE-OS 26 25 399 | 0,0.05 | 273 | 3 |
| | 0,01 | 273 | 9 |

Tabelle 2

Lösungen mit 50 Gew. % Formaldehyd und 0,60 Gew. % Methanol

| Stabilisator (Dodecamethylen-bismelamin) | Gehalt (%) | Lagertemperatur (K) | Lagerhaltbarkeit (d) |
|---|---|---|---|
| gem. Erfindung | 0,01 | 312 | > 30 |
| | 0,015 | 312 | > 60 |
| gem. EP-A2-79037 | 0,01 | 312 | 12 |
| | 0,015 | 312 | 30 |
| gem. DE-OS 26 25 399 | 0,01 | 312 | 1 |
| | 0,02 | 312 | 8 |

Herstellung von semiformalhaltigen Stabilisatorlösungen

Beispiel 5

40 kg Paraformaldehyd (90 Gew.-%) werden in 56 kg Methanol suspendiert und nach Zusatz von 0,5 kg eines Katalysators in Form eines organischen Amins, wie Triethylamin, Diethylamin, Pyridin, Hexamethylentetramin u.a., zum Sieden erhitzt. Nach ca. 20 min erhält man eine vollkommen klare Lösung, zu der man 24,1 kg des DBM zusetzt. Es wird weitere 30 min gerührt und gegebenenfalls von unlöslichen Nebenbestandteilen filtriert. Nach dem Erkalten ist die 20-Gew. %ige Stabilisatorlösung gebrauchsfertig.

D e g u s s a Aktiengesellschaft
Frankfurt am Main

Verfahren zur Herstellung von Dodecamethylenbismelamin und dessen Verwendung

Patentansprüche

1. Verfahren zur Herstellung von Dodecamethylenbismelamin durch Umsetzung von 2,4-Diamino-6-chlortriazin-(1,3,5) mit 1,12-Diaminododecan in einer alkalischen, wässrigen Suspension, dadurch gekennzeichnet, daß man 1,12-Diaminododecan in einem mit Wasser mischbaren, organischen Lösungsmittel gelöst der Suspension zusetzt, die Reaktion bei Rückflußtemperatur durchführt, anschließend das Reaktionsgemisch auf Raumtemperatur abkühlt und das auskristallisierte Produkt abtrennt.

2. Verfahren nach Anspruch 1, dadurch gekennzeichnet, daß man als Lösungsmittel einen aliphatischen Alkohol mit 1 bis 3-C-Atomen einsetzt.

3. Verfahren nach Anspruch 1, dadurch gekennzeichnet, daß man als Lösungsmittel Dioxan einsetzt.

4. Verfahren nach Anspruch 1, dadurch gekennzeichnet, daß man als Lösungsmittel Ethylenglykolmonomethylether einsetzt.

5. Verwendung von Dodecamethylenbismelamin hergestellt nach dem Verfahren gemäß den Ansprüchen 1 bis 4 zur Stabilisierung von wässrigen Formaldehydlösungen.

6. Verwendung von Dodecamethylenbismelamin hergestellt nach dem Verfahren gemäß den Ansprüchen 1 bis 4 oder durch Umsetzung von 2,4-Diamino-6-chlortriazin-(1,3,5) mit 1,12-Diaminododecan in einer wässrigen, lösungsmittelfreien oder wasserfreien, DMF-haltigen Suspension zur Stabilisierung von wässrigen Formaldehydlösungen, dadurch gekennzeichnet, das man das Bismelamin in Form einer 15 bis 25 Gew.%-igen Lösung einsetzt, bezogen auf die Gesamtmenge, wobei als Lösungsmittel Methoxymethanol (Methylsemiformal) in Form einer methanolischen Lösung verwendet wird, die einen Gehalt von 70 - 85 % an Methoxymethanol aufweist.